(19)

**Europäisches Patentamt**
**European Patent Office**
**Office européen des brevets**

(11) **EP 4 434 446 A1**

(12) **EUROPEAN PATENT APPLICATION**

(43) Date of publication:
**25.09.2024  Bulletin 2024/39**

(21) Application number: **24164573.8**

(22) Date of filing: **19.03.2024**

(51) International Patent Classification (IPC):
***A61B 5/00*** *(2006.01)*

(52) Cooperative Patent Classification (CPC):
**A61B 5/0075; A61B 5/443; A61B 5/4875;**
**A61B 5/6802; A61B 5/7239; A61B 5/7278;**
A61B 2562/043

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **23.03.2023  US 202363454226 P**
**01.03.2024  US 202418593532**

(71) Applicant: **Apple Inc.**
**Cupertino, CA 95014 (US)**

(72) Inventors:
• **CIVITCI, Fehmi**
**Cupertino, 95014 (US)**
• **NAYAK, Aditya B.**
**Cupertino, 95014 (US)**
• **LU, Dawei**
**Cupertino, 95014 (US)**
• **LI, Haokun**
**Cupertino, 95014 (US)**

(74) Representative: **COPA Copenhagen Patents**
**Rosenørns Allé 1, 2nd floor**
**1970 Frederiksberg C (DK)**

(54) **AN OPTICAL SENSING SYSTEM FOR DETERMINING BODY WATER CONTENT**

(57)     Embodiments are directed to an electronic device that includes a housing, a light emitter, a first detector positioned at a first separation distance from the light emitter, and a second detector positioned at a second separation distance from the light emitter. The electronic device can include a processor that is configured to cause the light emitter to emit light toward a user, receive a first measurement from the first detector based on a return of the emitted light from tissue of the user and receive a second measurement from the second detector based on a return of the emitted light from the tissue of the user. The processor can determine a decay constant of the emitted light using the first measurements and the second measurements, and determine a water content metric of the user using the determined decay constant.

*FIG. 1*

## Description

### CROSS-REFERENCE TO RELATED APPLICATION

This application is a nonprovisional and claims the benefit under 35 U.S.C. § 119(e) of U.S. Provisional Patent Application No. 63/454,226, filed March 23, 2023, the contents of which are incorporated herein by reference as if fully disclosed herein.

### FIELD

The described embodiments relate generally to systems and methods for determining body water content of a user and more particularly to systems and methods for determining body water content for a user.

### BACKGROUND

Wearable electronic devices are increasingly incorporating sensing systems that measure physiological parameters of a user. Water is a major component of the body and plays an important role in numerous physiological processes. Maintaining body water content is similarly important to a user's health and wellbeing, and a user's body water content may vary with hydration. Recommended body water content may vary between individuals based on a number of factors, such as a user's age, weight, and/or height. However, accurately determining body water content outside of clinical settings can be difficult.

### SUMMARY

Embodiments are directed to an electronic device including a housing, a light emitter, a first detector positioned at a first separation distance from the light emitter, and a second detector positioned at a second separation distance from the light emitter. The electronic device can also include a processor that is configured to cause the light emitter to emit light toward a user, receive first measurements from the first detector that represent a first return of the emitted light from the tissue of the user, and receive second measurements from the second detector that represent a second return of the emitted light from the tissue of the user. The processor can determine a decay constant of the emitted light using the first measurements and the second measurements and determine a water content metric of the user using the determined decay constant.

Embodiments are also directed to an electronic device that includes a first light emitter configured to emit first light at a first wavelength, a second light emitter configured to emit second light at a second wavelength, and multiple light detectors including at least a first light detector and a second light detector. The electronic device can include a processor configured to receive first measurements from the first light detector based on a return of emitted first light and the emitted second light from a user and receive second measurements from the second light detector based on a return of the emitted first light and emitted second light from the user. The processor can determine a decay constant of the emitted light using the first measurements and the second measurements, and determine a water content metric of the user using the determined decay constant.

Embodiments are further directed to methods for estimating a body water content of a user. The methods can include emitting light at a first wavelength from one or more light emitters and towards skin of a user, measuring a first return of the emitted light that has interacted with the tissue of a user, the first return of the emitted light associated with a first light emitter and light detector pair having a first separation distance, and measuring a second return of the emitted light that has interacted with the tissue of the user, the second return of the emitted light associated with a second light emitter detector pair having a second separation distance. The methods can include determining, by a processor, a decay constant of the emitted light using the measured first return of the emitted light and the measured second return of the emitted light and determining, by the processor, a water content metric of the user using the decay constant.

### BRIEF DESCRIPTION OF THE DRAWINGS

The disclosure will be readily understood by the following detailed description in conjunction with the accompanying drawings, wherein like reference numerals designate like structural elements, and in which:

FIG. 1 shows a functional block diagram of an example electronic device that may be used to perform optical measurements for determining water content of a user as described herein;

FIG. 2 shows an example process for performing optical measurements to determine water content of a user;

FIG. 3 shows an example light emitter and light detector configuration for performing optical measurements to determine water content of a user;

FIG. 4 shows a visual example of measurement data that can be used to determine a decay constant from light measurements made by multiple detectors;

FIG. 5 shows an example process for performing optical measurements to determine water content of a user;

FIGs. 6A-6B show example light emitter and detector configurations for performing optical measurements to determine water content of a user, as described herein; and

FIG. 7 shows a block diagram of an example electronic device that may incorporate a sensing assembly as described herein.

## DETAILED DESCRIPTION

Reference will now be made in detail to representative embodiments illustrated in the accompanying drawings. It should be understood that the following descriptions are not intended to limit the embodiments to one preferred embodiment. To the contrary, it is intended to cover alternatives, modifications, and equivalents as can be included within the spirit and scope of the described embodiments as defined by the appended claims.

Embodiments disclosed herein are directed to methods and devices that can be used to determine body water content of a user. The methods may include determining body water content of a user based on changes in light that have interacted with tissue of the user (e.g., skin tissue including the epidermis, dermis, hypodermis and/or other subcutaneous tissue). In some cases, light can be emitted toward a user and one or more light detectors can measure a return of the emitted light that has interacted with the user. The wavelength(s) and/or other properties of the emitted light can be selected so that the light is absorbed (or otherwise changed) based on the body water content of the user. For example, the water content in a user's skin may affect absorption, scattering and/or other properties of the emitted light. The measured light that is returned from the user's skin can be used to determine the body water content of the user. In some cases, the amplitude of the light after it has interacted with the skin can be measured and used to determine a body water content for a user. For example, the amplitude of the light may decrease based on the amount of water in the skin of the user.

The term "body water content" is used herein to refer the amount of water that is contained within a user's body (e.g., water in the tissues, blood, bones and elsewhere). The water content metric may be used to express the body water content of a person as a percentage of a person's total mass, or other suitable unit of measure. For example, a water content metric can indicate that a person is 60% water by weight. In some cases, the water content metric may be based on the measured amount of water in a specific tissue (e.g., skin and/or other subcutaneous tissues), and the body water content can be determined from measuring the amount of water in a specific tissue (e.g., the skin and/or other subcutaneous tissues) and estimating the total amount of water in a user's body (e.g., water in blood, bones and other tissues) based on the skin measurements.

In other instances, the water content metric may be a relative value that is calculated as a relative difference between the user's body water content as currently measured and a target value. The water content metric may be expressed, for example, as a percentage deviation from the target value. In some variations the target value may be selected from or otherwise determined from one or more previous body water content measurements. In these instances, the target value may act as a baseline for the user, and the water content metric may indicate how close a user is to their baseline (e.g., 2% above baseline, 3% below baseline, and so on). In some of these variations, a user's baseline (and thus the target value used in determining the water content metric) may vary over time depending on the user's body water content. In other variations, the target value may be a fixed value that represents a target body water content for a user. In these instances, the water content metric may indicate how close a user is to their target body water content (e.g., 1% above target body water content, 2% below target body water content, and so on). Different target values may be selected, if so desired, as the target body water content for a user changes (e.g., depending on changes to the anticipated activity levels of a user).

In some cases, an exponential function can be fit to the measured light data and parameters of the fit can be used to determine a water content metric that characterizes or estimates the body water content of a user. The use of the exponential function may increase the accuracy and/or repeatability of determining body water content of a user. For example, emitted light may be measured at multiple detectors each located at a different distance from the light source. An exponential function may be fit to the measured light data which may include information on how an amplitude of the light decreases based on the travel distance through the tissue (e.g., due to absorption, scattering and so on). The exponential function, determined from the measured light data, can include a decay constant that characterizes the change in amplitude of light (or other property of the light) based on the distance that the light traveled through the tissue of the user. The decay constant can be correlated to a body water content of the user.

Using multiple light paths to determine a decay constant for emitted light based on its interaction with the body may help increase the accuracy or repeatability by reducing the measurement sensitivity to the intensity of the emitted light as compared to ratio-metric measurements which may compare the absorption of signals to determine a desired parameter. That is, determining a decay constant for the light may require less precise control over the intensity of the emitted light, because the decay constant is based on relative values of the emitted light. Accordingly, the intensity of the emitted light may have less significance when the data points for a given data set are collected using the same emitted light or under similar conditions. Additionally or alternatively, depending on the number of different data sets, there may be a benefit to using multiple light paths which may allow measurement noise to be averaged out. For example, using multiple light paths to determine a decay constant may also allow occlusion artifacts, such as pigment changes, scars, hair, or other skin discontinuities, to be removed from the measurement data.

In some cases, an electronic device for measuring body water content can include one or more light emitters and multiple light detectors each positioned at a different separation distance from the one or more light emitters. The electronic device can emit light from the one or more light emitters and into tissue of the user. For example, the electronic device can emit light into the skin of the user. The electronic device can measure light that has interacted with the tissue at each of the multiple light detectors. The measured light can be used to determine a decay constant of the light based on the light's interactions with the user's tissue. The decay constant can be used to determine body water content of the user. For example, a decay constant for a particular light source (e.g., a particular wavelength of light) may be correlated to body water content of a user. Accordingly determining the decay constant of the emitted light that has interacted with the tissue of the user can be used to determine a body water content of the user.

In some cases, the electronic device may include multiple light emitters, which may have different positions with respect to the light detectors and/or emit different wavelengths of light. A decay constant can be determined for light emitted from each of the different light emitters. In some cases, if multiple light emitters emit light at the same wavelength, each of the transmission paths between the different light emitters and light detectors may provide data points that can be used to fit the exponential function to the extent that one of the emitters has a different separation distance to at least one of the light detectors. Additionally or alternatively, multiple measurements from light emitter and light detector pairs and/or from a single light emitter and light detector pair may be averaged or otherwise combined to form the measurement value of the data point. If the multiple light emitters each emit light at different wavelengths, decay constants may be determined for each wavelength of light. The decay constants for each of the different wavelengths of light may be correlated to body water content so that the decay constant determined from the measured light can be used to determine body water content. In some cases, the decay constants for different wavelengths of light can be averaged or otherwise analyzed to determine a single body water content for the user.

In some cases, the system may be able to measure (or use) a skin pigmentation of a user, which can be used to determine the body water content from the decay constant. The skin pigmentation can be determined from the same sensors used to determine the decay constant or from a separate set of sensors. The interaction of the light with the skin - for example, the absorption and/or scatter of the light by the skin - may depend on the pigmentation of the skin. Accordingly, measuring and/or characterizing the pigmentation of the skin of the user may be used to select a correlation between the decay constant and body water content. For example, the electronic device can be configured with different correlations between the decay constant and body water content for different pigmentations of skin. Additionally or alternatively, the system may track skin pigmentation of the user over time, which may be analyzed to determine changes in the skin pigmentation.

In some cases, the system may analyze different light paths (e.g., light that has traveled from an emitter, through the tissue and to a detector) to determine whether a suitable measurement can be obtained. The system may be configured to analyze different light emitter and light detector pairs to determine if there is an occlusion (e.g., a portion of a user's tissue that includes a scab, hair, scar tissue, dust, or other contaminants, and so on) that blocks or absorbs a significant amount of light that would otherwise travel between the emitter and the detector.

In some cases, the electronic devices described herein can include wearable electronic devices such as a smartwatch, headband, in/over ear devices such as headphones, be incorporated into clothing or include any other suitable wearable devices. The electronic devices described herein can also include portable or other devices that a user may contact for a period of time during a body water content measurement. For example, the electronic device may include a smartphone and a user may contact a portion of the smartphone to take a body water content measurement. Additionally, the techniques described herein may be used with other physiological measurement systems integrated into the electronic device and/or associated wearable device. For example, body water content measurements may also use or be analyzed using electrocardiograph (ECG) measurements, photoplethysmography (PPG) measurements, hear rate monitors, blood oxygen saturation monitors, and so on.

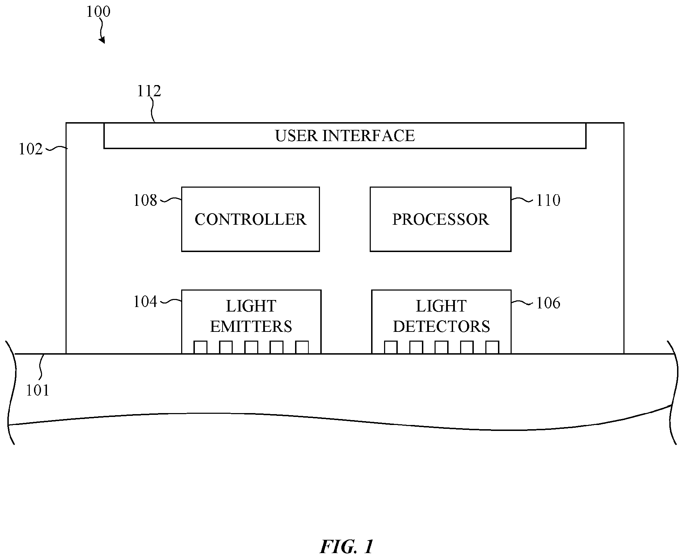

These and other embodiments are discussed below with reference to FIGs. 1-7. However, those skilled in the art will readily appreciate that the detailed description given herein with respect to these Figures is for explanatory purposes only and should not be construed as limiting.

FIG. 1 shows a functional block diagram of an example electronic device that may be used to perform optical measurements for determining water content of a user as described herein. The electronic device 100 can include one or more light emitters 104 that are configured to emit light into tissue of a user 101, and one or more light detectors 106 that are configured to measure a return of the emitted light from the tissue. The electronic device 100 can also include a controller 108, a processor 110, and a user interface 112. The electronic device 100 may include a housing 102 and the light emitters 104, the light detectors 106, the controller 108, the processor 110, and the user interface 112 may be positioned at least partially within the housing 102.

The light emitters 104 can include any suitable light source including light emitting diodes (LEDs), laser light sources, and so on. The light emitters 104 can be positioned on and/or in the housing 102 such that the light emitters 104 emit light toward a user (e.g., a user's skin, subcutaneous tissue, and so on) when the electronic device 100 is worn by the user 101. In some cases, one or more components including lenses, polarizing components, and/or filters can be positioned between the light emitters 104 and the user 101. In some cases, these components (e.g., a lens, a polarizer, and/or other optical components) can be configured to increase an intensity of light that is transmitted into the tissue of the user 101. The light emitters 104 can be positioned in a variety of arrangements on the housing 102 of the electronic device 100 and/or within the electronic device 100. In some cases, the light emitters 104 may also be used for other functions in addition to body water content measurements. For example, the light emitters 104 can be used to perform other physiological measurements.

The light emitters 104 can include emitters that are configured to emit light at one or more wavelengths. In some cases, the wavelength(s) of light emitted by the light emitters 104 can be selected based on the specific wavelength(s) interaction with water in the skin or other tissue of the user 101. For example, the light emitters 104 may be configured to emit light at wavelengths that are scattered, absorbed, or otherwise modified based on the amount of water in the tissue. In some cases, the light emitters 104 can include multiple emitters and different emitters may emit light at different wavelengths. Accordingly, multiple wavelengths of light may be used to determine body water content of a user 101.

The light detectors 106 can include any suitable light detectors that are configured to sense light emitted by the light emitters 104 and that has passed through the tissue of the user 101. The light detectors 106 can be positioned on and/or in the housing 102 such that the light detectors 106 can measure light that was emitted from the one or more light emitters 104 and has interacted with the tissue of the user 101. In some cases, one or more components including lenses, polarizing components, and/or filters can be positioned between the light detectors 106 and the user 101. In some cases, these components (e.g., a lens, a polarizer and/or other optical components) can be configured to increase an intensity of light that is transmitted from the user 101 and to the light detectors 106. Additionally or alternatively, various ones of the detectors can be configured to measure specific wavelengths of light that are emitted from the emitter(s).

In some cases, the controller 108 can operate the light emitters 104 and/or light detectors 106 to emit light into tissue of the user 101 and measure the emitted light after it has interacted with the tissue of the user 101. The controller 108 can be configured to drive the light emitters 104 and light detectors 106. In some cases, the controller 108 may control when individual light emitters 104 emit light and one or more light detectors 106 that are used to measure the emitted light. For example, the controller 108 may cause different wavelengths of light to be emitted at different times.

The controller 108 may be operably coupled to the processor 110. In some cases, all or part of the controller 108 may be implemented as computer-executable code executing on the processor 110. In some cases, all or part of the controller 108 may include or be implemented by dedicated hardware, including hardware for selectively operating electrodes in drive and/or sensor modes. In some cases, the controller 108 may include dedicated processing units for carrying out one or more functions of the electronic device 100 as described herein.

The user interface 112 may include a display, a touch-sensitive display, speakers, microphones, haptic engines, or any other suitable input/output devices. The user interface may include components that are positioned within an interior of the electronic device 100, components that are partially within a housing of the electronic device 100 (e.g., a display) and/or components that are separate from and operably coupled to the electronic device 100 (e.g., headphones).

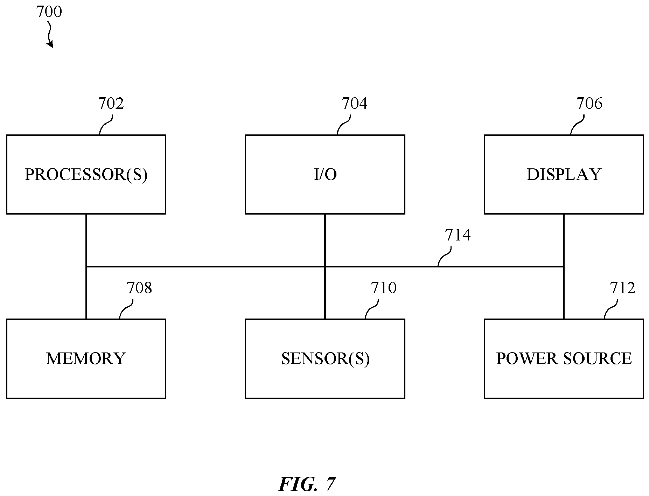

The electronic device 100 may include additional and/or alternative components that are not shown in FIG. 1. For example, the electronic device 100 may include input devices, output devices, memory, a power source, and/or electrical connectors that operably couple the components of the electronic device 100. Example components of the wearable electronic device 100 are discussed in more detail with respect to FIG. 7. Example electronic devices may include smartphones, tablets, portable music players or other portable electronic devices; wearable electronic devices as described herein include a smartwatch.

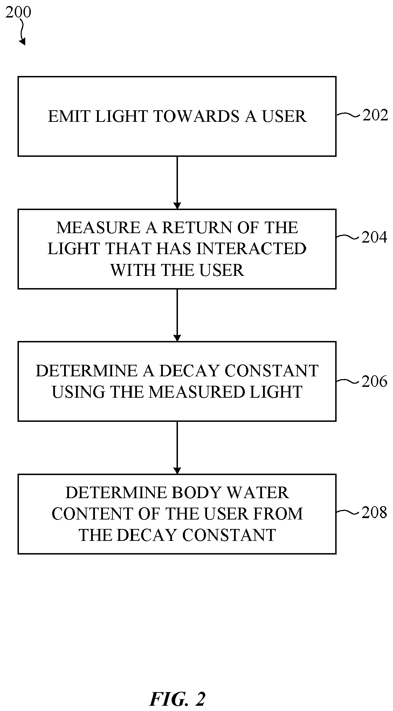

FIG. 2 shows an example process 200 for performing optical measurements to determine water content of a user. The process 200 can be performed by the devices described herein including electronic device 100.

At operation 202, the process 200 can include emitting light towards a user. Light can be emitted from one or more light emitters. In some cases, multiple light emitters may emit light at the same wavelength. Additionally or alternatively, the one or more light emitters may emit light at different wavelengths. In cases where the system has multiple light emitters, the emission from different emitters may occur at different times, for example, when the same detector is used with multiple different light emitters or when light emitters of different wavelengths are used. In some cases, the light emitters can be configured to emit light into a tissue of the user which may include using lenses, polarizers, or other optical components that contact the user and receive light from the light emitter and cause the light to be transmitted into the tissue. Light transmitted into the tissue may interact with the tissue including being absorbed by, scattered by, or otherwise modified by the tissue including water in the tissue.

The parameters of light (e.g., wavelength) used in performing body water content measurements may be selected to have a certain amount of absorption by water. For example, water in the tissue may absorb and/or scatter emitted light based on the wavelength of the light. Accordingly, the system may use one or more wavelengths of light that allow a decay constant to be determined from the measured light and a body water content of the user to be determined. Additionally or alternatively, an amplitude ratio(s) between different wavelengths of light can eb used to determine one or more decay constants.

At operation 204, the process 200 can include measuring a return of the emitted light that has interacted with the tissue of the user. In some cases, an electronic device can include one or more light detectors that measure light that is emitted from the light emitters, has passed through tissue of the user, and returned to the electronic device (e.g., via scattering within the tissue). The light detectors may be positioned at different separation distances from the one or more light emitters. Different light detectors may measure light that has traveled different distances through the tissue of the user. Each light detector measurement may be associated with a different separation distance depending on whether the emitter(s) are active during a measurement.

The light detectors may be configured to measure light at a specific wavelength or range of wavelengths, which may correspond to a wavelength(s) of light emitted by the one or more emitters. In some cases, the detectors may include lenses, filters (e.g., polarizers), or other optical components that optically couple the detectors to the user and transmit light from the tissue of the user and to the detectors.

At operation 206, the process 200 can include determining a decay constant of an exponential function using the light measured by the detectors. The light detectors may be positioned with respect to the one or more light emitters to facilitate fitting of an exponential function to the measured light data where the exponential function characterizes the interaction of the light with the tissue. The exponential function may be used to characterize the absorption, scattering, and/or other interactions with the tissue. In some cases, the exponential function can include a decay constant that at least partially characterizes how the intensity, amplitude, or other property of light decreases as a result of interacting with the tissue. An example exponential function is given by Equation 1:

$$ S = ae^{-\tau d} + b \qquad (1) $$

In Equation 1, S is the measurement of the signal value, a is the light amplitude of the emitted light, $\tau$ is the decay constant, d is the separation distance between the light source and the detector, and b is the offset which may be due to ambient light or other factors.

The positioning of the detectors with respect to the one or more light emitters may be used to fit an exponential function that characterizes the interaction of light with the tissue. For example, multiple light detectors may each be positioned at a different separation distance from a light emitter. The light measurement signal value at each detector (at each different separation distance) may be used to fit an exponential function. For example, the parameters of the exponential function shown in Equation (1) may be adjusted to determine a best fit of the exponential function to the light measurement signal values at the different locations of the detectors. Accordingly, the fitting of the exponential function to the measured light signal values may include determining a decay constant $\tau$ for the emitted light, which characterizes a decrease in the light signal as it travels through the tissue.

At operation 208, the process can include determining body water content of the user from the decay constant. The correlation between the decay constant $\tau$ and body water content can be done experimentally or using any other suitable techniques. In some cases, the system may use a water content metric to indicate the body water content of the user. For example, the water content metric may be a water percentage of the user's total mass. In some cases, the water content metric indicates a relative body water content change of the user, which may be a change in water content with respect to baseline or a target water content value, as described herein. In some cases, the water content metric may be determined from a local measured of water content (e.g., water content of the skin of the user). For example, the water content metric can be a percentage of water in the skin (or other tissue) of the user. In other cases, the water content metric can be a global estimate of body water content (e.g., estimated total body water content of the user). The global estimate of the body water content may be determined from the local measured water content (e.g., amount of water in the skin and/or other tissue). For example, the system may use a look-up table or other relational database that provides a correlation between the measured local water content and the global body water content of a user. This correlation may take various factors into account including age, weight, height, and so on.

In some cases, the correlation between the decay constant $\tau$ and body water content can be based on a variety of factors including age, body composition (e.g., weight, height, body mass index (BMI), body surface area (BSA), and so on), and/or any other suitable physiological parameters of a user. Accordingly, an electronic device can be configured to use a correlation between the decay constant $\tau$ and body water content of a user based on one or more user specific parameters, which may be input, stored, or otherwise determined by the electronic device.

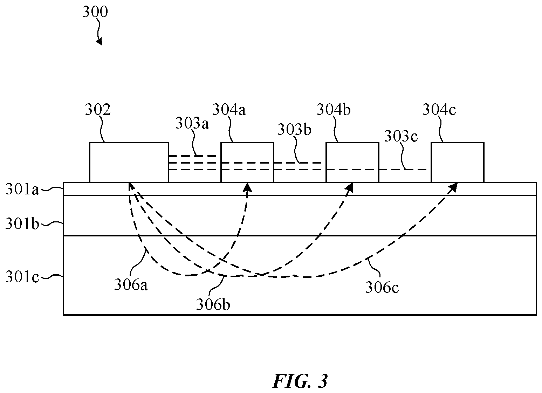

FIG. 3 shows an example light emitter and light detector configuration 300 for performing optical measurements to determine water content of a user. The light emitter and light detector configuration 300 can be incorporated into an electronic device (e.g., electronic device 100) as described herein and include a light emitter 302 and multiple light detectors 304. The light emitter and light detector configuration 300, provides an example to illustrate the concepts described herein and the concepts described with respect to the light emitter and light detector configuration 300 can be applied to other configurations including electronic devices that have multiple light emitters and multiple light detectors, light emitters and light detectors arranged in different configurations, and so on.

The light emitter 302 can be configured to emit light 306 into tissue 301 of a user. In some cases, the tissue 301 can include skin tissue of the user, which may have multiple tissue layers that the emitted light 306 interacts with. For example, the tissue 301 may include a first tissue layer 301a which corresponds to the epidermis, a second tissue layer 301b corresponding to the dermis, and a third tissue layer 301c corresponding to the hypodermis or other subcutaneous tissue layers. The emitted light 306 shows example light paths that travel through the tissue 301 and are detected by the light detectors 304 for the sake of clarity. The light emitter can emit light that travels many different paths including shallower and deeper depths into the tissue, in other directions and so on. Accordingly, the light detectors 304 may only measure a portion of the emitted light 306.

Each light detector 304 can be positioned at a different separation distance from the light emitter 302. For example, a first light detector 304a may be positioned at a first distance 303a from the light emitter 302, the second light detector 304b may be positioned at a second distance 303b that is greater than the first distance, and the third light detector 304c may be positioned at a third distance 303c that is greater than the second distance. Accordingly, the first light detector 304a may primarily measure first light 306a that has traveled the shortest distance through the tissue, the second light detector 304b may primarily measure second light 306b that has traveled a greater distance than the first light 306a, and the third light detector 304c may primality measure third light 306c that has traveled the greatest distance of the light emitters 302.

The distance between the light emitter 302 and each light detector 304 can be correlated to the measurements made by a respective light detector 304. For example, an electronic device may be configured with defined distances between the light emitter 302 and each light detector 304. Measurements made by the first light detector 304a can be correlated with the distance between the first light detector 304a and the light emitter 302. Measurements made by the second light detector 304b can be correlated with the distance between the second light detector 304b and the light emitter 302. Measurements made by the third light detector 304c can be correlated with the distance between the third light detector 304c and the light emitter 302. The measurements of the emitted light 306 made by the light detectors 304 and the relative (and/or defined) distances between each light detector 304 and the emitter can be used to fit an exponential function and determine a decay constant as described herein.

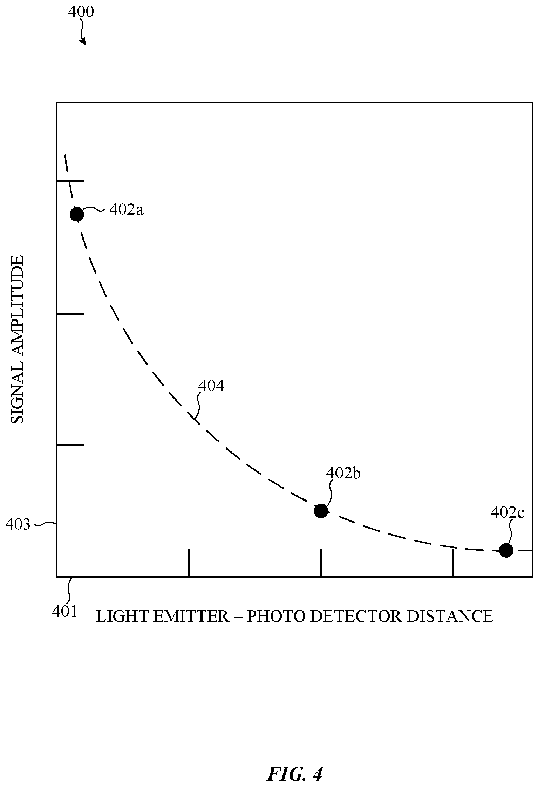

FIG. 4 shows a visual example of measurement data 400 that can be used to determine a decay constant from light measurements made by multiple detectors. The measurement data 400 is an example of measurement data from the example light emitter and light detector configuration 300 shown in FIG. 3.

The measurement data 400 can include light measurements 402 made by the light detectors 304. The measurement data 400 is shown in graphical form where a first axis 401 represents the distance between a light emitter and the light detector and the second axis 403 represents the signal amplitude of emitted light that has interacted with tissue of the user and been measured by a detector.

The measurement data 400 may include one or more first measurements 402a which correspond to light amplitudes measured at a first detector (e.g., the first light detector 304a) having a first separation distance from the light detector. The measurement data may include one or more second measurements 402b which correspond to light amplitudes measured at a second detector (e.g., the second light detector 304b) having a second separation distance from the light emitter. The measurement data may also include one or more third measurements 402c which correspond to light amplitudes measured at a third detector (e.g., the third light detector 304c) having a third separation distance from the light emitter. In some cases, the exponential function (e.g., the exponential function shown in Equation 1) may be fit using the first measurements 402a, the second measurements 402b, and the third measurements 402c.

An exponential function 404 can be fit to the light measurements 402. For example, the parameters of the exponential function shown in Equation 1 can be determined from fitting the exponential function to the light measurements 402. In some cases, the exponential function can be fit to the light measurements 402 using any suitable curve fitting process. In some cases, the light measurements data may be filtered, averaged, or otherwise pre-processed prior to fitting the data. Additionally or alternatively, fitting processing may include smoothing operations, interpolation, or any other suitable techniques.

In some cases, the exponential function (e.g., the exponential function shown in Equation 1) may be fit using the first measurements 402a and the second measurements 402b. In some cases, the exponential function (e.g., the exponential function shown in Equation 1) may be fit using the first measurements 402a, the second measurements 402b, and the third measurements 402c.

The decay constant $\tau$ can then be determined from the exponential function shown in Equation 1. The body water content of a user can be determined from a correlation between the decay constant $\tau$ to body water content of a user as described herein.

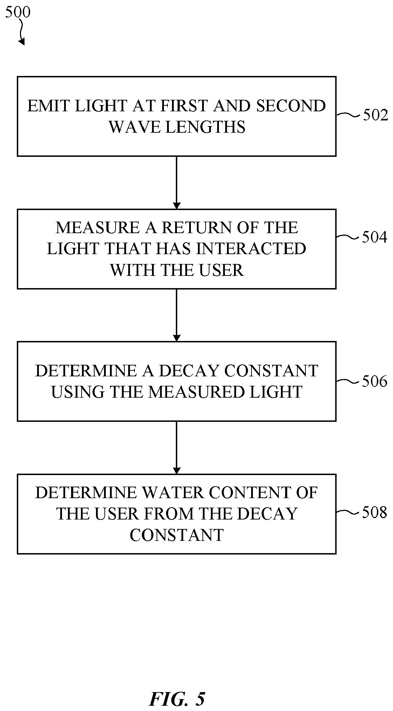

FIG. 5 shows an example process 500 for performing optical measurements to determine water content of a user. The process 500 may be performed by the electronic devices described herein (e.g., electronic device 100). The process 500 may emit multiple different wavelengths of light into tissue of a user to determine body water content of a user. In some cases, a decay constant may be determined for each wavelength of light and body water content may be determined from multiple decay constants. Additionally or alternatively, one or more wavelengths of light may be used to calibrate or select correlations between measured light and body water content. For example, one or more wavelengths of light may be used to determine a skin pigment of a user and the fitting of the exponential function and/or the correlation between the decay constant and body water content may be based on the determined skin pigment.

At operation 502, the process 500 can include emitting light at a first wavelength (or range of wavelengths) from a first light emitter and into tissue of the user. Operation 502 can also include emitting light at a second wavelength (or range of wavelengths), which are different from the first wavelength, from a second light emitter and into tissue of the user. In some cases, the first wavelength may be selected based on an interaction with water in the tissue of the user. For example, the first and/or second wavelengths may be selected based on its scattering, or absorption based on the amount of water in the tissue, such that measuring a decrease in the amplitude of the light at different distances can be used to determine water content as described herein. Additionally or alternatively, the first and/or second wavelengths of light may be selected based on an interaction of the light to different pigments of tissue. For example, a wavelength may be selected based on a correlation between the interaction with the pigment and a property of the measured light (e.g., decrease in the amplitude of the light).

At operation 504, the process 500 can include measuring a return of the emitted light that has passed through tissue of the user. In some cases, each of the first and second wavelengths of light can be measured at multiple separation distances from a respective emitter as described herein. For example, the first wavelength of light may be measured by multiple detectors each positioned at a different separation distance from the first emitter. Additionally or alternatively, the second wavelength of light may be measured by multiple detectors each positioned at a different separation distance from the second emitter. In some cases, the same detectors can be used to measure the first and second wavelengths of light. In other cases, different sets of detectors can be used to measure the first and second wavelengths of light and the different sets of detectors may have the same or different separation distances from the respective light emitters.

At operation 506, the process 500 can include determining a decay constant using the measured light.

In some cases, a decay constant can be determined for each wavelength of light. For example, a first decay constant can be determined from fitting an exponential function to the light measurements of the first wavelength of light. A second decay constant can be determined from fitting an exponential function to the light measurements of the second wavelength of light. In some cases, multiple measurements may be made at the detectors for each wavelength of light, and the multiple measurements for each wavelength may be filtered, averaged, or otherwise processed to reduce noise, remove outlies, and/or remove other sources of error from the data.

At operation 508, the process 500 can include determining water content of the user from the multiple decay constants. In some cases, the decay constant for a given wavelength of light may be dependent on both the wavelength of light and the water content in the tissue. Accordingly, the correlation between the decay constant and water content may also be dependent on the wavelength. For example, the operation 508 may include using a first set of correlation data for the first wavelength of light to determine a first body water content for the first wavelength. Operation 508 may also include using a second set of correlation data for the second wavelength of light to determine a second body water content for the user. The first and second body water measurements may be averaged or otherwise combined to determine an overall body water content for the user. In some cases, this may include weighting a body water content determined from one of the wavelengths more strongly, which may be based on the retaliative interaction of the different light with water in the tissue, an uncertainty associated with each measurement or other suitable factors.

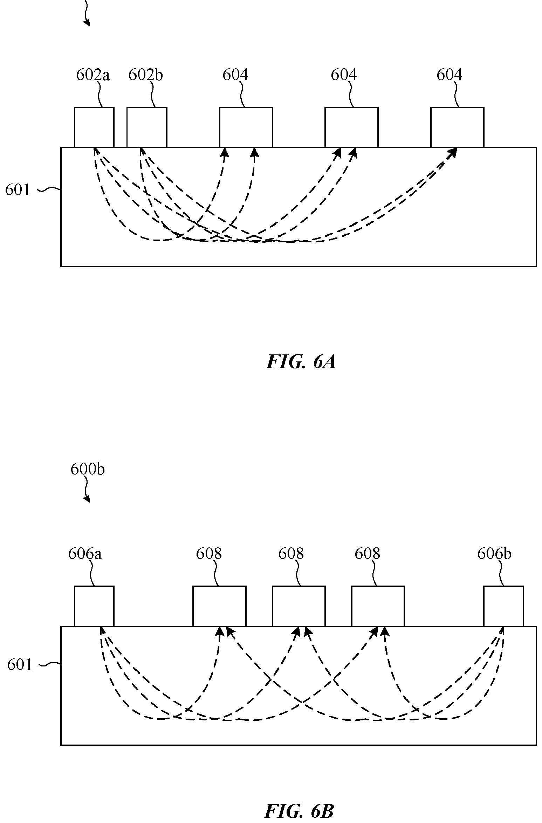

FIGs. 6A-6B show example light emitter and detector configurations 600 for performing optical measurements to determine water content of a user, as described herein. The example light emitter configurations 600 can be implemented using an electronic device that includes light emitters and detectors including the electronic devices described herein (e.g., electronic device 100).

FIG. 6A shows a first light emitter and detector configuration 600a that includes multiple emitters 602 and multiple detectors 604. The light emitters 602 can include a first light emitter 602a that emits light at one or more first wavelengths and into tissue 601. The light emitters 602 can also include a second light emitter 602b that emits light at one or more second wavelengths, different from the first wavelength(s), and into tissue 601. In some cases, the detectors 604 can use the same detectors to measure emitted light from the first light emitter 602a and the second light emitter 602b. In other cases, different detectors 604 may be used to measure different wavelengths of light.

FIG. 6B shows a second light emitter and detector configuration 600b that includes one or more first light emitters 606a positioned on a first side of light detectors 608 and one or more second light emitters 606b positioned on a second side of the light detectors 608.

In some cases, the first light emitters 606a and the second light emitters 606b may include emitters that emit light at the same wavelength(s). In other cases, the first light emitters 606a and the second light emitters 606b may emit light at one or more different wavelengths.

In some cases, the light emitter(s) and/or light detector(s) can be arranged as a multi-dimensional array (e.g., a 2-dimensioal array). Light from a light emitter may be measured by multiple detectors in the array, where each of the different detectors have a different spatial arraignment (e.g., separation distance and location) with respect to the light emitter. Additionally or alternatively, multiple light emitters can be arranged in an array and various emitters may emit light at the same or different wavelengths. Different light emitters or sets of light emitters (e.g., associated with different wavelengths of light) may emit light at different times, which may be measured at one or more detectors. The spatial relationship between the light emitters and array of detectors may be used to fit an exponential function to the measured light data as described herein. Additionally or alternatively, the spatial arrangement of the array of light detectors with respect to the light emitter(s) may be used to correct for alignment or other measurement error.

FIG. 7 is an example block diagram of a body water content measurement system 700, which can take the form of any of the devices as described with references to FIGs. 1-6B. The body water content measurement system 700 can include a processor 702, an input/output (I/O) mechanism 704 (e.g., wired or wireless communications interfaces), a display 706, memory 708, sensors 710 (e.g., physiological sensors such as those described herein), and a power source 712 (e.g., a rechargeable battery). The processor 702 can control some or all of the operations of the body water content measurement system 700. The processor 702 can communicate, either directly or indirectly, with some or all of the components of the body water content measurement system 700. For example, a system bus or other communication mechanism 714 can provide communication between the processor 702, the I/O mechanism 704, the memory 708, the sensors 710, and the power source 712.

The processor 702 can be implemented as any electronic device capable of processing, receiving, or transmitting data or instructions. For example, the processor 702 can be a microprocessor, a central processing unit (CPU), an application-specific integrated circuit (ASIC), a digital signal processor (DSP), or combinations of such devices. As described herein, the term "processor" is meant to encompass a single processor or processing unit, multiple processors, multiple processing units, or other suitable computing element or elements. The processing unit can be programmed to perform the various aspects of the systems described herein.

It should be noted that the components of the body water content measurement system 700 can be controlled by multiple processors. For example, select components of the body water content measurement system 700 (e.g., a sensor 710) may be controlled by a first processor and other components of the body water content measurement system 700 (e.g., the I/O 704) may be controlled by a second processor, where the first and second processors may or may not be in communication with each other.

The I/O device 704 can transmit and/or receive data from a user or another electronic device. An I/O device can transmit electronic signals via a communications network, such as a wireless and/or wired network connection. Examples of wireless and wired network connections include, but are not limited to, cellular, Wi-Fi, Bluetooth, IR, and Ethernet connections. In some cases, the I/O device 704 can communicate with an external electronic device, such as a smartphone, electronic device, or other portable electronic device, as described here.

The body water content measurement system 700 may optionally include a display 706 such as a liquid-crystal display (LCD), an organic light emitting diode (OLED) display, a light emitting diode (LED) display, or the like. If the display 706 is an LCD, the display 706 may also include a backlight component that can be controlled to provide variable levels of display brightness. If the display 706 is an OLED or LED type display, the brightness of the display 706 may be controlled by modifying the electrical signals that are provided to display elements. The display 706 may correspond to any of the displays shown or described herein.

The memory 708 can store electronic data that can be used by the body water content measurement system 700. For example, the memory 708 can store electrical data or content such as, for example, audio and video files, documents and applications, device settings and user preferences, timing signals, control signals, and data structures or databases. The memory 708 can be configured as any type of memory. By way of example only, the memory 708 can be implemented as random access memory, read-only memory, Flash memory, removable memory, other types of storage elements, or combinations of such devices.

The body water content measurement system 700 may also include one or more sensors 710 positioned almost anywhere on the body water content measurement system 700. The sensor(s) 710 can be configured to sense one or more types of parameters, such as but not limited to, pressure, light, touch, heat, movement, relative motion, biometric data (e.g., biological parameters), and so on. For example, the sensor(s) 710 may include a heat sensor, a position sensor, a light or optical sensor, an accelerometer, a pressure transducer, a gyroscope, a magnetometer, a health monitoring sensor, and so on. Additionally, the one or more sensors 710 can utilize any suitable sensing technology, including, but not limited to, capacitive, ultrasonic, resistive, optical, ultrasound, piezoelectric, and thermal sensing technology.

The power source 712 can be implemented with any device capable of providing energy to the body water content measurement system 700. For example, the power source 712 may be one or more batteries or rechargeable batteries. Additionally or alternatively, the power source 712 can be a power connector or power cord that connects the body water content measurement system 700 to another power source, such as a wall outlet.

As described above, one aspect of the present technology is measuring physiological conditions of a user such as determining body water content of a user and the like. The present disclosure contemplates that in some instances this gathered data may include personal information data that uniquely identifies or can be used to contact or locate a specific person. Such personal information data can include demographic data, location-based data, telephone numbers, email addresses, Twitter IDs (or other social media aliases or handles), home addresses, data or records relating to a user's health or level of fitness (e.g., vital signs measurements, medication information, exercise information), date of birth, or any other identifying or personal information.

The present disclosure recognizes that the use of such personal information data, in the present technology, can be used to the benefit of users. For example, the personal information data can be used to provide haptic or audiovisual outputs that are tailored to the user. Further, other uses for personal information data that benefit the user are also contemplated by the present disclosure. For instance, health and fitness data may be used to provide insights into a user's general wellness or may be used as positive feedback to individuals using technology to pursue wellness goals.

The present disclosure contemplates that the entities responsible for the collection, analysis, disclosure, transfer, storage, or other use of such personal information data will comply with well-established privacy policies and/or privacy practices. In particular, such entities should implement and consistently use privacy policies and practices that are generally recognized as meeting or exceeding industry or governmental requirements for maintaining the privacy and security of personal information data. Such policies should be easily accessible by users and should be updated as the collection and/or use of data changes. Personal information from users should be collected for legitimate and reasonable uses of the entity and not shared or sold outside of those legitimate uses. Further, such collection/sharing should occur after receiving the informed consent of the users. Additionally, such entities should consider taking any needed steps for safeguarding and securing access to such personal information data and ensuring that others with access to the personal information data adhere to their privacy policies and procedures. Further, such entities can subject themselves to evaluation by third parties to certify their adherence to widely accepted privacy policies and practices. In addition, policies and practices should be adapted for the particular types of personal information data being collected and/or accessed and revised to adhere to applicable laws and standards, including jurisdiction-specific considerations. For instance, in the US, collection of or access to certain health data may be governed by federal and/or state laws, such as the Health Insurance Portability and Accountability Act ("HIPAA"); whereas health data in other countries may be subject to other regulations and policies and should be handled accordingly. Hence different privacy practices should be maintained for different personal data types in each country.

Despite the foregoing, the present disclosure also contemplates embodiments in which users selectively block the use of, or access to, personal information data. That is, the present disclosure contemplates that hardware and/or software elements can be provided to prevent or block access to such personal information data. For example, in the case of determining spatial parameters, the present technology can be configured to allow users to select to "opt in" or "opt out" of participation in the collection of personal information data during registration for services or anytime thereafter. In addition to providing "opt in" and "opt out" options, the present disclosure contemplates providing notifications relating to the access or use of personal information. For instance, a user may be notified upon downloading an app that their personal information data will be accessed and then reminded again just before personal information data is accessed by the app.

Moreover, it is the intent of the present disclosure that personal information data should be managed and handled in a way to minimize risks of unintentional or unauthorized access or use. Risk can be minimized by limiting the collection of data and deleting data once it is no longer needed. In addition, and when applicable, including in certain health related applications, data de-identification can be used to protect a user's privacy. De-identification may be facilitated, when appropriate, by removing specific identifiers (e.g., date of birth, etc.), controlling the amount or specificity of data stored (e.g., collecting location data at a city level rather than at an address level), controlling how data is stored (e.g., aggregating data across users), and/or other methods.

Therefore, although the present disclosure broadly covers use of personal information data to implement one or more various disclosed embodiments, the present disclosure also contemplates that the various embodiments can also be implemented without the need for accessing such personal information data. That is, the various embodiments of the present technology are not rendered inoperable due to the lack of all or a portion of such personal information data. For example, haptic outputs may be provided based on non-personal information data or a bare minimum amount of personal information, such as events or states at the device associated with a user, other non-personal information, or publicly available information.

The foregoing description, for purposes of explanation, used specific nomenclature to provide a thorough understanding of the described embodiments. However, it will be apparent to one skilled in the art that the specific details are not required in order to practice the described embodiments. Thus, the foregoing descriptions of the specific embodiments described herein are presented for purposes of illustration and description. They are not targeted to be exhaustive or to limit the embodiments to the precise forms disclosed. It will be apparent to one of ordinary skill in the art that many modifications and variations are possible in view of the above teachings.

Exemplary embodiments of the present disclosure are set out in the following items:

1. An electronic device comprising:

a housing;
a light emitter;
a first detector positioned at a first separation distance from the light emitter;
a second detector positioned at a second separation distance from the light emitter; and
a processor configured to:

cause the light emitter to emit light toward tissue of a user;
receive a first measurement from the first detector that represents a first return of the emitted light from the tissue of the user;
receive a second measurement from the second detector that represents a second return of the emitted light from the tissue of the user;
determine a decay constant of the emitted light using the first measurement and the second measurement; and
determine a water content metric of the user using the determined decay constant.

2. The electronic device of item 1, wherein:

determining the decay constant comprises fitting an exponential function to the first measurement and the second measurement; and
the decay constant indicates an exponential decay of the emitted light as a function of the first separation distance and the second separation distance.

3. The electronic device of any of items 1-2, wherein:

the processor is further configured to:

determine a first signal amplitude using the first measurement; and
determine a second signal amplitude using the second measurement; and

the decay constant is determined using the first signal amplitude and the second signal amplitude.

4. The electronic device of any of items 1-3, wherein:

the light emitter is a first light emitter configured to emit first light;
the electronic device comprises a second light emitter configured to emit second light;
the first detector is positioned at a third separation distance from the second light emitter; and
the second detector is positioned at a fourth separation distance from the second light emitter.

5. The electronic device of item 4, wherein:

the decay constant is a first decay constant; and
the processor is configured to:

receive a third measurement from the first detector that represents a first return of the emitted second light from the tissue of the user;
receive a fourth measurement from the second detector that represents a second return of the emitted second light from the tissue of the user;
determine a second decay constant of the second emitted light using the first measurement and the second measurement; and
determine the water content metric of the user using the first and second decay constants.

6. The electronic device of any of items 4-5, wherein:

the first light emitter is configured to emit the first light at a first wavelength; and
the second light emitter is configured to emit the second light at a second wavelength different from the first wavelength.

7. The electronic device of any of items 1-6, further comprising a third detector positioned at a third separation distance from the light emitter, wherein the processor is configured to:

receive a third measurement from the third detector that represents a third return of the emitted light from the tissue of the user; and
determine the decay constant of the emitted light using the third measurement.

8. The electronic device of any of items 1-7, wherein:

the light emitter is a first light emitter configured to emit first light;

the electronic device comprises a second light emitter configured to emit second light; and the processor is configured:

to characterize a skin pigmentation of the user using at least one of the first light or the second light; and determine the decay constant of the emitted light based on the skin pigmentation.

9. An electronic device comprising:

a first light emitter configured to emit first light at a first wavelength toward a user; a second light emitter configured to emit second light at a second wavelength toward the user; an array of light detectors comprising at least a first light detector and a second light detector; and a processor configured to:

receive a first measurement from the first light detector based on a return of the emitted first light and the emitted second light from the user; receive a second measurement from the second light detector based on the return of the emitted first light and emitted second light from the user; determine a decay constant of emitted light using the first measurement and the second measurement; and determine a water content metric of the user using the determined decay constant.

10. The electronic device of item 9, wherein:

the array of light detectors comprises a third light detector; and the processor is configured to:

receive a third measurement from the third light detector based on the return of the emitted first light and the emitted second light from the user; and determine the decay constant of the emitted light using the third measurement.

11. The electronic device of item 10, wherein each of the first light detector, the second light detector, and the third light detector are located at different separation distances from at least one or more of the first light emitter or the second light emitter.

12. The electronic device of any of items 9-11, further comprising a third light emitter configured to emit third light at the first wavelength, wherein:

the first light emitter is positioned on a first side the array of light detectors; and the third light emitter is positioned on a second side of the array of light detectors.

13. The electronic device of item 12, wherein the processor is configured to:

receive a third measurement from the first light detector based on a return of the emitted third light and the emitted second light from the user; and select one of the first measurement or the third measurement to determine the decay constant based on comparing the first measurement and the second measurement.

14. The electronic device of any of items 9-13, wherein determining the decay constant comprises fitting an exponential function to the first measurement and the second measurement.

15. The electronic device of any of items 9-14, further comprising a housing, wherein the first light emitter, the second light emitter, and the array of light detectors are each positioned at least partially within the housing.

16. A method for estimating a body water content of a user, the method comprising:

emitting light at a first wavelength from one or more light emitters and towards skin of a user; measuring a first return of the emitted light that has interacted with tissue of the user, the first return of the emitted light associated with a first light emitter and light detector pair having a first separation distance; measuring a second return of the emitted light that has interacted with the tissue of the user, the second return of the emitted light associated with a second light emitter and detector pair having a second separation distance; determining, by a processor, a decay constant of the emitted light using the measured first return of the emitted light and the measured second return of the emitted light; and determining, by the processor, a water content metric of the user using the decay constant.

17. The method of item 16, further comprising:

measuring a third return of the emitted light that has interacted with the tissue of the user at a third light emitter and light detector pair having a third separation distance; and determining the decay constant using the measured third return of the emitted light.

18. The method of item 17, further comprising:

emitting second light a second wavelength from the one or more light emitters towards the skin of the user;
measuring a first return of the second light that has interacted with the tissue of the user at a fourth light emitter and light detector pair having a fourth separation distance;
measuring a second return of the second light that has interacted with the tissue of the user at a fifth light emitter and light detector pair having a fifth separation distance; and
determining a decay constant of the emitted second light using the measured first return of the second light and the measured second return of the second light; and
determining the water content metric of the user using the decay constant.

19. The method of item 18, further comprising:

measuring a third return of the second light that has interacted with the tissue of the user at a sixth light emitter and light detector pair having a sixth separation distance; and
determining the decay constant using the measured third return of the emitted light.

20. The method of any of items 18-19, wherein the first light emitter and light detector pair is the same as the fourth light emitter and light detector pair.

**Claims**

1. An electronic device comprising:

a housing;
a light emitter;
a first detector positioned at a first separation distance from the light emitter;
a second detector positioned at a second separation distance from the light emitter; and
a processor configured to:

cause the light emitter to emit light toward tissue of a user;
receive a first measurement from the first detector that represents a first return of the emitted light from the tissue of the user;
receive a second measurement from the second detector that represents a second return of the emitted light from the tissue of the user;
determine a decay constant of the emitted light using the first measurement and the second measurement; and

determine a water content metric of the user using the determined decay constant.

2. The electronic device of claim 1, wherein:

determining the decay constant comprises fitting an exponential function to the first measurement and the second measurement; and
the decay constant indicates an exponential decay of the emitted light as a function of the first separation distance and the second separation distance.

3. The electronic device of any of claims 1-2, wherein:

the processor is further configured to:

determine a first signal amplitude using the first measurement; and
determine a second signal amplitude using the second measurement; and

the decay constant is determined using the first signal amplitude and the second signal amplitude.

4. The electronic device of any of claims 1-3, wherein:

the light emitter is a first light emitter configured to emit first light;
the electronic device comprises a second light emitter configured to emit second light;
the first detector is positioned at a third separation distance from the second light emitter; and
the second detector is positioned at a fourth separation distance from the second light emitter.

5. The electronic device of claim 4, wherein:

the decay constant is a first decay constant; and
the processor is configured to:

receive a third measurement from the first detector that represents a first return of the emitted second light from the tissue of the user;
receive a fourth measurement from the second detector that represents a second return of the emitted second light from the tissue of the user;
determine a second decay constant of the second emitted light using the first measurement and the second measurement; and
determine the water content metric of the user using the first and second decay constants.

6. The electronic device of any of claims 4-5, wherein:

the first light emitter is configured to emit the first light at a first wavelength; and
the second light emitter is configured to emit the second light at a second wavelength different from the first wavelength.

7. The electronic device of any of claims 1-6, further comprising a third detector positioned at a third separation distance from the light emitter, wherein the processor is configured to:

receive a third measurement from the third detector that represents a third return of the emitted light from the tissue of the user; and
determine the decay constant of the emitted light using the third measurement.

8. The electronic device of any of claims 1-7, wherein:

the light emitter is a first light emitter configured to emit first light;
the electronic device comprises a second light emitter configured to emit second light; and
the processor is configured:

to characterize a skin pigmentation of the user using at least one of the first light or the second light; and
determine the decay constant of the emitted light based on the skin pigmentation.

9. A method for estimating a body water content of a user, the method comprising:

emitting light at a first wavelength from one or more light emitters and towards skin of a user;
measuring a first return of the emitted light that has interacted with tissue of the user, the first return of the emitted light associated with a first light emitter and light detector pair having a first separation distance;
measuring a second return of the emitted light that has interacted with the tissue of the user, the second return of the emitted light associated with a second light emitter and detector pair having a second separation distance;
determining, by a processor, a decay constant of the emitted light using the measured first return of the emitted light and the measured second return of the emitted light; and
determining, by the processor, a water content metric of the user using the decay constant.

10. The method of claim 9, further comprising:

measuring a third return of the emitted light that has interacted with the tissue of the user at a third light emitter and light detector pair having a third separation distance; and
determining the decay constant using the measured third return of the emitted light.

11. The method of claim 10, further comprising:

emitting second light a second wavelength from the one or more light emitters towards the skin of the user;
measuring a first return of the second light that has interacted with the tissue of the user at a fourth light emitter and light detector pair having a fourth separation distance;
measuring a second return of the second light that has interacted with the tissue of the user at a fifth light emitter and light detector pair having a fifth separation distance; and
determining a decay constant of the emitted second light using the measured first return of the second light and the measured second return of the second light; and
determining the water content metric of the user using the decay constant.

12. The method of claim 11, further comprising:

measuring a third return of the second light that has interacted with the tissue of the user at a sixth light emitter and light detector pair having a sixth separation distance; and
determining the decay constant using the measured third return of the emitted light.

13. The method of any of claims 11-12, wherein the first light emitter and light detector pair is the same as the fourth light emitter and light detector pair.

100

112

USER INTERFACE

102

108 CONTROLLER

PROCESSOR 110

104 LIGHT EMITTERS

LIGHT DETECTORS 106

101

*FIG. 1*

200

EMIT LIGHT TOWARDS A USER ~202

MEASURE A RETURN OF THE
LIGHT THAT HAS INTERACTED
WITH THE USER ~204

DETERMINE A DECAY CONSTANT
USING THE MEASURED LIGHT ~206

DETERMINE BODY WATER
CONTENT OF THE USER FROM
THE DECAY CONSTANT ~208

*FIG. 2*

**FIG. 3**

400

FIG. 4

500

```
┌─────────────────────────────┐
│  EMIT LIGHT AT FIRST AND SECOND  │ ～502
│       WAVE LENGTHS          │
└─────────────────────────────┘
            │
            ▼
┌─────────────────────────────┐
│    MEASURE A RETURN OF THE     │ ～504
│  LIGHT THAT HAS INTERACTED    │
│        WITH THE USER         │
└─────────────────────────────┘
            │
            ▼
┌─────────────────────────────┐
│  DETERMINE A DECAY CONSTANT    │ ～506
│    USING THE MEASURED LIGHT    │
└─────────────────────────────┘
            │
            ▼
┌─────────────────────────────┐
│  DETERMINE WATER CONTENT OF    │ ～508
│   THE USER FROM THE DECAY     │
│          CONSTANT           │
└─────────────────────────────┘
```

*FIG. 5*

**FIG. 6A**

**FIG. 6B**

700

702

706

704

PROCESSOR(S)

I/O

DISPLAY

714

708

710

712

MEMORY

SENSOR(S)

POWER SOURCE

*FIG. 7*

Europäisches
Patentamt

European
Patent Office

Office européen
des brevets

## EUROPEAN SEARCH REPORT

**Application Number**

EP 24 16 4573

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 894 520 A2 (SAMSUNG ELECTRONICS CO LTD [KR]) 5 March 2008 (2008-03-05)<br>* paragraph [0019] – paragraph [0021] *<br>* paragraph [0027] *<br>* paragraph [0044] – paragraph [0047] *<br>* paragraph [0057] *<br>* paragraph [0068] – paragraph [0079] *<br>* figures 1-5,7,8 *<br>----- | 1-13 | INV.<br>A61B5/00 |
| A | US 10 206 619 B1 (LEE JANICE C [US] ET AL) 19 February 2019 (2019-02-19)<br>* column 4, line 12 – line 26 *<br>* column 12, line 49 – line 67 *<br>* column 16 *<br>* equation 4 *<br>* figure 12D *<br>----- | 1-13 | |
| A | US 2018/249937 A1 (WIESE DANIEL [US] ET AL) 6 September 2018 (2018-09-06)<br>* figure 1B *<br>* paragraph [0047] *<br>* paragraph [0091] *<br>----- | 1-13 | |
| A | CN 101 581 666 A (UNIV HUAZHONG SCIENCE TECH [CN]) 18 November 2009 (2009-11-18)<br>* figure 3 *<br>* paragraph [0023] – paragraph [0030] *<br>----- | 1-13 | **TECHNICAL FIELDS SEARCHED (IPC)**<br><br>A61B<br>G01N |
| A | US 2019/374134 A1 (CHEN YU-WEN [TW] ET AL) 12 December 2019 (2019-12-12)<br>* paragraph [0021] *<br>----- | 1-13 | |
| A | KR 2021 0035714 A (UNIV DANKOOK CHEONAN CAMPUS IND ACADEMIC COOPERATION FOUNDATION [KR]) 1 April 2021 (2021-04-01)<br>* paragraph [0051] *<br>----- | 1-13 | |

-/--

The present search report has been drawn up for all claims

| Place of search | Date of completion of the search | Examiner |
|---|---|---|
| The Hague | 26 April 2024 | Costa Angeli, M |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

Europäisches
Patentamt

European
Patent Office

Office européen
des brevets

**EUROPEAN SEARCH REPORT**

Application Number

**EP 24 16 4573**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2019/137385 A1 (NAPPEZ THOMAS [FR] ET AL) 9 May 2019 (2019-05-09) * paragraph [0084] - paragraph [0105] * ----- | 1-13 | |

TECHNICAL FIELDS
SEARCHED      (IPC)

The present search report has been drawn up for all claims

| Place of search | Date of completion of the search | Examiner |
|---|---|---|
| The Hague | 26 April 2024 | Costa Angeli, M |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 16 4573

This annex lists the patent family members relating to the patent documents cited in the above-mentioned European search report.
The members are as contained in the European Patent Office EDP file on
The European Patent Office is in no way liable for these particulars which are merely given for the purpose of information.

26-04-2024

| Patent document cited in search report | | Publication date | Patent family member(s) | | Publication date |
|---|---|---|---|---|---|
| EP 1894520 | A2 | 05-03-2008 | CN 101133951 | A | 05-03-2008 |
| | | | EP 1894520 | A2 | 05-03-2008 |
| | | | JP 4718531 | B2 | 06-07-2011 |
| | | | JP 2008055163 | A | 13-03-2008 |
| | | | KR 20080020340 | A | 05-03-2008 |
| | | | US 2008058617 | A1 | 06-03-2008 |
| US 10206619 | B1 | 19-02-2019 | NONE | | |
| US 2018249937 | A1 | 06-09-2018 | AU 2017226296 | A1 | 20-09-2018 |
| | | | EP 3422944 | A1 | 09-01-2019 |
| | | | JP 2019512364 | A | 16-05-2019 |
| | | | US 2018249937 | A1 | 06-09-2018 |
| | | | US 2019099116 | A1 | 04-04-2019 |
| | | | WO 2017151856 | A1 | 08-09-2017 |
| CN 101581666 | A | 18-11-2009 | NONE | | |
| US 2019374134 | A1 | 12-12-2019 | CN 110575138 | A | 17-12-2019 |
| | | | EP 3581096 | A1 | 18-12-2019 |
| | | | TW 202000130 | A | 01-01-2020 |
| | | | US 2019374134 | A1 | 12-12-2019 |
| KR 20210035714 | A | 01-04-2021 | NONE | | |
| US 2019137385 | A1 | 09-05-2019 | EP 3449239 | A1 | 06-03-2019 |
| | | | FR 3050824 | A1 | 03-11-2017 |
| | | | US 2019137385 | A1 | 09-05-2019 |
| | | | WO 2017187088 | A1 | 02-11-2017 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63454226